# EUROPEAN PATENT APPLICATION

(11) **EP 4 768 572 A1**
(43) Date of publication of application: **01.07.2026**
(21) Application number: 24856483.3
(22) Date of filing: 21.08.2024
(51) Int. Cl.: C12N 5/075, C12N 5/10, C12N 5/076, C12N 5/0735, C12N 15/09

(54) **METHOD FOR PRODUCING OOGONIUM OR PRO-SPERMATOGONIUM**

(30) Priority: 21.08.2023 JP 2023133928
(71) Applicant: Kyoto University, Kyoto-shi, Kyoto 606-8501 (JP)
(72) Inventor: SAITOU, Mitinori, Kyoto-shi, Kyoto 606-8501 (JP); MURASE, Yusuke, Kyoto-shi, Kyoto 606-8501 (JP); YOKOGAWA, Ryuta, Kyoto-shi, Kyoto 606-8501 (JP)
(74) Representative: Gill Jennings & Every LLP
(86) International application number: PCT/JP2024/029671
(87) International publication number: WO 2025/041795

(57) **Abstract**

An object of the present invention is to provide a method for simply and efficiently inducing differentiation of oogonia or prospermatogonia from primordial germ cell-like cells or primordial germ cells. The object is solved by a method for producing oogonia or prospermatogonia comprising a step of culturing primordial germ cell-like cells or primordial germ cells in a medium containing a BMP receptor type 2 signal activator to obtain oogonia or prospermatogonia.

## Description

### Technical Field

The present invention relates to a method for producing oogonia or prospermatogonia, comprising a step of inducing differentiation of oogonia or prospermatogonia from primordial germ cell-like cells (PGCLCs) or primordial germ cells (PGCs), and to a cell population containing oogonia or prospermatogonia obtained by said method.

### Background Art

In reproductive medicine such as infertility treatment, the production of germ cells (sperm and eggs) in vitro is attracting attention. As the differentiation mechanism of germ cells is becoming clearer, the development of technologies to produce germ cells from pluripotent stem cells (such as embryonic stem (ES) cells and induced pluripotent stem (iPS) cells) is progressing.

Non-Patent Document 1 describes that cynomolgus monkey ES cells were differentiated into primordial germ cell-like cells (PGCLCs) by culturing them in a medium containing BMP4, SCF, LIF, EGF, and a ROCK inhibitor. However, under these culture conditions as they are, it is not possible to further differentiate PGCLCs into oogonia or prospermatogonia. To differentiate PGCLCs into oogonia or prospermatogonia, it is necessary to mix PGCLCs with xenogeneic cells or cells derived from other individuals, such as mouse ovarian cells or testicular cells, and culture them in a manner that provides the PGCLCs with a special external environment that promotes induction of differentiation into germ cells. For example, Non-Patent Document 2 describes that ES cells are differentiated into PGCLCs by culturing in a medium containing BMP4, SCF, LIF, and EGF, and the obtained PGCLCs can be differentiated into spermatogonia by adding a cAMP activator (forskolin), a PDE4 inhibitor (rolipram), and an immunosuppressant/cell death inhibitor (cyclosporin A) to the medium, and mixing and culturing them with mouse testicular cells.

On the other hand, Patent Document 1 discloses a method for inducing mouse PGCLCs or PGCs into oocytes in the presence of BMP and retinoic acid without co-culture with ovarian cells or spermatogonia.

However, for human PGCLCs or PGCs, a method for inducing differentiation into oogonia or prospermatogonia without co-culture with xenogeneic ovarian cells or testicular cells, or ovarian cells or testicular cells derived from other individuals, has not been disclosed.

### Prior Art Documents

### Patent Documents

Patent Document 1: WO 2019/107576

### Non-Patent Documents

Non-Patent Document 1: EMBO J. 2023 May 2;42(9):e112962.
Non-Patent Document 2: Cell Stem Cell. 2021 Dec 2;28(12):2167-2179.e9.

### Summary of Invention

### Problems to be Solved by the Invention

As described above, the current situation is that in order to differentiate PGCLCs or PGCs into oogonia or prospermatogonia, they must be co-cultured with xenogeneic ovarian cells or testicular cells, or ovarian cells or testicular cells derived from other individuals, in order to provide the PGCLCs or PGCs with an external environment for differentiation induction. Therefore, even if oogonia or prospermatogonia are induced to differentiate by conventional methods, these oogonia and prospermatogonia are obtained in a mixed state with xenogeneic ovarian cells or testicular cells, or ovarian cells or testicular cells derived from other individuals, and thus do not result in a pure cell population. That is, a technology for differentiating PGCLCs or PGCs into oogonia or prospermatogonia while maintaining a pure cell population has not yet been established. Accordingly, an object of the present invention is to provide a method for simply and efficiently inducing the differentiation of oogonia or prospermatogonia from PGCLCs or PGCs, even without providing an external environment that promotes differentiation induction into oogonia or prospermatogonia using xenogeneic ovarian cells or testicular cells, or ovarian cells or testicular cells derived from other individuals, and further to provide a method for simply and efficiently inducing the differentiation of a cell population containing oogonia or prospermatogonia with high purity from PGCLCs or PGCs.

### Means for Solving the Problems

The inventors of the present invention have conducted intensive studies to solve the above problems. As a result, they have found that by culturing PGCLCs or PGCs in a medium containing a BMP receptor type 2 signal activator, it is possible to simply and efficiently induce differentiation into oogonia or prospermatogonia even without providing an external environment that promotes differentiation induction into oogonia or prospermatogonia using xenogeneic ovarian cells or testicular cells, or ovarian cells or testicular cells derived from other individuals, thereby completed the present invention.

The gist of the present invention is as follows.
[1] A method for producing oogonia or prospermatogonia, comprising a step of culturing primordial germ cell-like cells or primordial germ cells in a medium containing a BMP receptor type 2 signal activator to obtain oogonia or prospermatogonia.
[2] The method for producing oogonia or prospermatogonia according to [1], wherein the BMP receptor type 2 signal activator is one or more selected from the group consisting of BMP2, BMP4, and BMP7.
[3] The method for producing oogonia or prospermatogonia according to [1] or [2], wherein the medium further contains a Wnt signal inhibitor.
[4] The method for producing oogonia or prospermatogonia according to [3], wherein the Wnt signal inhibitor is IWR1 or XAV939.
[5] The method for producing oogonia or prospermatogonia according to any one of [1] to [4], wherein the medium further contains one or a combination of two or more selected from stem cell factor (SCF), a cAMP enhancer, and basic fibroblast growth factor (bFGF).
[6] The method for producing oogonia or prospermatogonia according to any one of [1] to [5], wherein the culture period is 10 days or more.
[7] The method for producing oogonia or prospermatogonia according to any one of [1] to [6], wherein the oogonia and prospermatogonia are DAZL and DDX4 positive cells.
[8] The method for producing oogonia or prospermatogonia according to any one of [1] to [7], wherein the primordial germ cell-like cells are primordial germ cell-like cells obtained by inducing differentiation of pluripotent stem cells.
[9] The method for producing oogonia or prospermatogonia according to any one of [1] to [8], which does not include a step of providing an external environment that promotes differentiation induction into oogonia or prospermatogonia using xenogeneic ovarian cells or testicular cells, or ovarian cells or testicular cells derived from other individuals.
[10] The method for producing oogonia or prospermatogonia according to any one of [1] to [9], wherein the primordial germ cell-like cells and primordial germ cells are human primordial germ cell-like cells and human primordial germ cells, and the oogonia and prospermatogonia are human oogonia and human prospermatogonia.
[11] A cell population obtained by inducing differentiation of primordial germ cell-like cells or primordial germ cells, comprising 80% or more of oogonia or prospermatogonia.

### Brief Description of Drawings

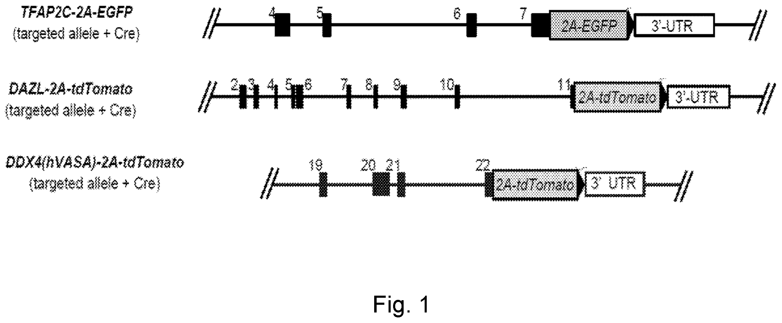

[Fig. 1] A diagram schematically showing TFAP2C-p2A-eGFP (AG), DAZL-p2A-tdTomato (DT), and DDX4-p2A-tdTomato (VT) reporter alleles.

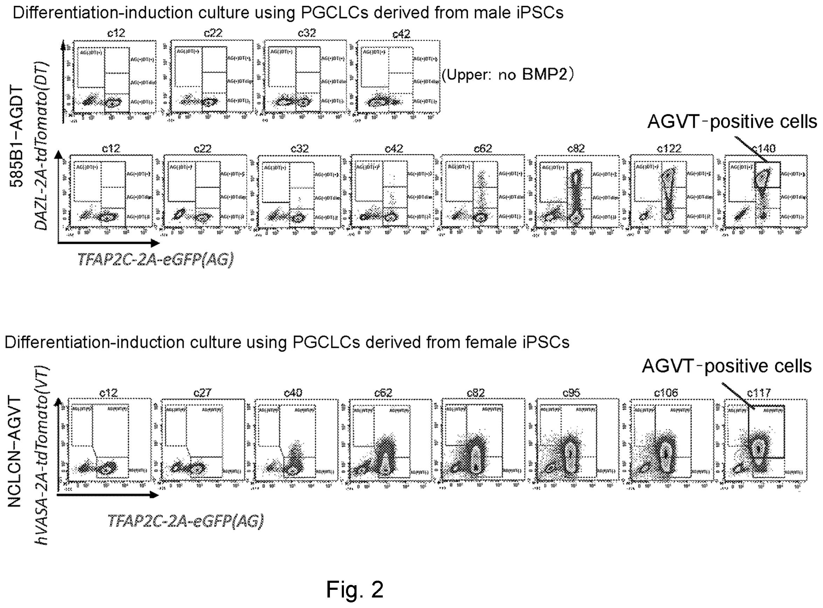

[Fig. 2] Graphs showing the analysis of fluorescence intensity of DAZL-p2A-tdTomato (DT) (vertical axis) and TFAP2C-p2A-eGFP (AG) (horizontal axis) in 585B1-AGDT hPGCLCs cultured in a medium for differentiation induction to oogonia or prospermatogonia from which only BMP2 was excluded, from day 12 to day 42 (c12 to c42) from the start of culture (Upper row: no BMP2); graphs showing the analysis of fluorescence intensity of DT (vertical axis) and AG (horizontal axis) in 585B1-AGDT hPGCLCs cultured in a medium for differentiation induction to oogonia or prospermatogonia (containing BMP2), from day 12 to day 140 (c12 to c140) from the start of culture (Middle row); and graphs showing the analysis of fluorescence intensity of VT (vertical axis) and AG (horizontal axis) in NCLCN-AGVT hPGCLCs cultured in a medium for differentiation induction to oogonia or prospermatogonia (containing BMP2), from day 12 to day 117 (c12 to c117) from the start of culture (Lower row).

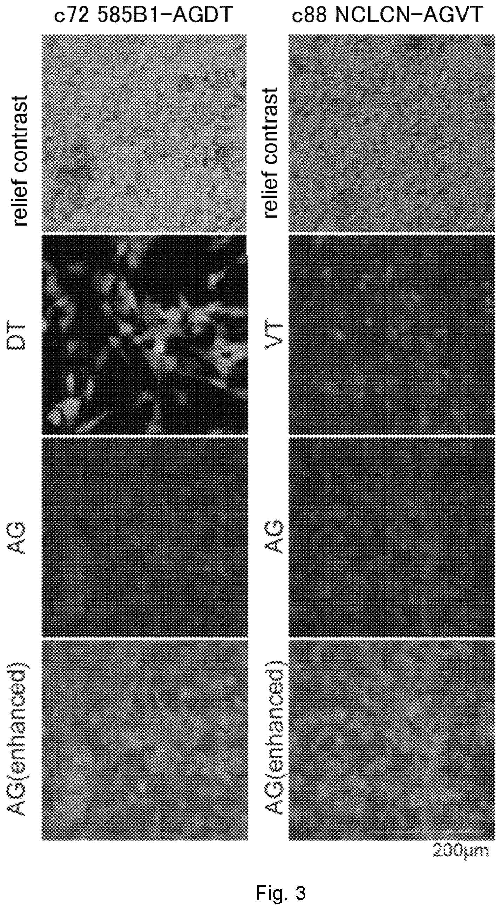

[Fig. 3] Photographs observing AG (green fluorescence), DT or VT (red fluorescence), and relief contrast with a microscope for 585B1-AGDT hPGCLCs on day 72 (c72 585B1-AGDT) and NCLCN-AGVT hPGCLCs on day 88 (c88 NCLCN-AGVT) after culture in a medium for differentiation induction to oogonia or prospermatogonia (drawing-substituting photographs). The scale bar represents 200 µm.

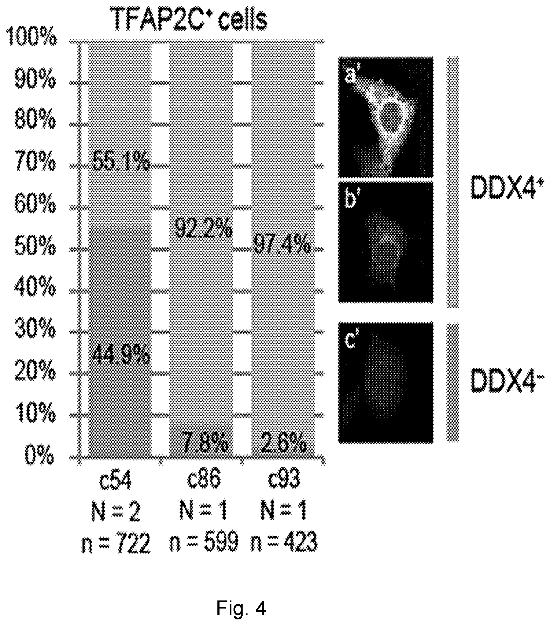

[Fig. 4] A diagram showing the ratio of DDX4-positive cells (DDX4+: upper section of the graph) and DDX4-negative cells (DDX4-: lower section of the graph) in TFAP2C-positive cells (TFAP2C+ cells) on day 54 (c54), day 86 (c86), and day 93 (c93) after culturing 1383D6 hiPSC-derived hPGCLCs in a medium for differentiation induction to oogonia or prospermatogonia (Left figure), and a diagram showing photographs obtained by immunostaining DDX4+ (a' and b') and DDX4- (c') respectively with DDX4 (drawing-substituting photograph: Right figure).

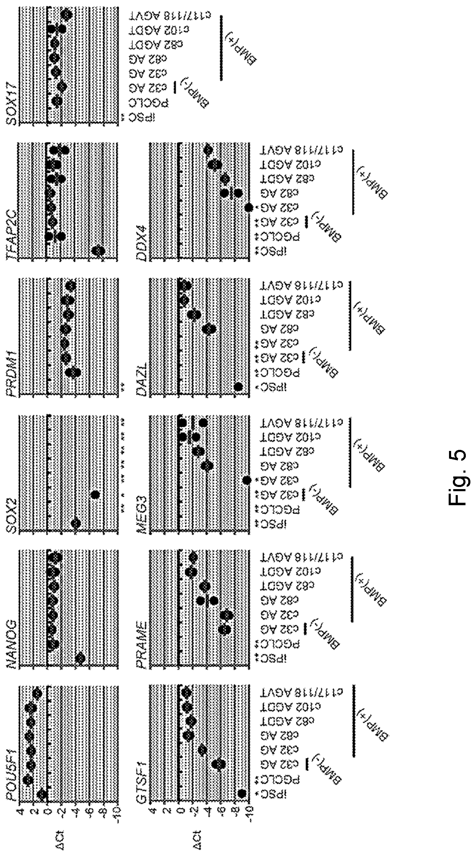

[Fig. 5] A diagram showing the results of analyzing the gene expression levels of POU5F1, NANOG, SOX2, PRDM1, TFAP2C, SOX17, GTSF1, PRAME, MEG3, DAZL, and DDX4 by qPCR for hiPSCs, hPGCLCs, 585B1-AGDT hPGCLCs cultured for 32 days in a medium for differentiation induction to oogonia or prospermatogonia from which only BMP2 was excluded (BMP(-): c32 AG), 585B1-AGDT hPGCLCs cultured in a medium for differentiation induction to oogonia or prospermatogonia (containing BMP2) for 32 days (AG+DT- cells) (BMP(+): c32 AG), for 82 days (AG+DT- cells) (BMP(+): c82 AG), for 82 days (AG+DT+ cells) (BMP(+): c82 AGDT), for 102 days (AG+DT+ cells) (BMP(+): c102 AGDT), and NCLCN-AGVT hPGCLCs cultured in a medium for differentiation induction to oogonia or prospermatogonia (containing BMP2) for 117 or 118 days (AG+VT+ cells) (BMP(+): c117/118 AGVT).

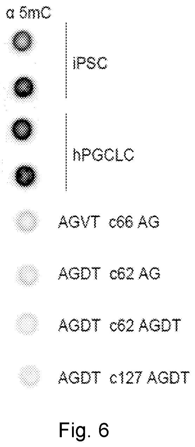

[Fig. 6] A diagram analyzing the 5mC level by DNA dot blot method for hiPSCs, hPGCLCs, AG+VT- cells on day 66 obtained by culturing NCLCN-AGVT hPGCLCs in a medium for differentiation induction to oogonia or prospermatogonia (AGVT c66 AG), AG+DT- cells on day 62 (AGDT c62 AG), AG+DT+ cells on day 62 (AGDT c62 AGDT), and AG+DT+ cells on day 127 (AGDT c127 AGDT) obtained by culturing 585B1-AGDT hPGCLCs in a medium for differentiation induction to oogonia or prospermatogonia.

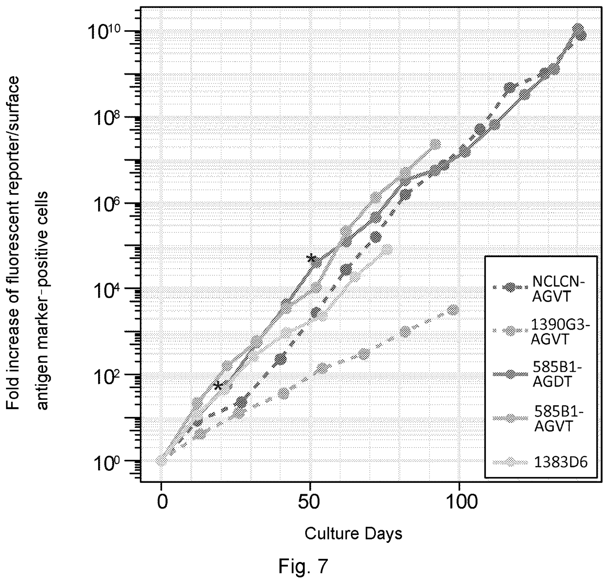

[Fig. 7] A diagram showing the results of culturing NCLCN-AGVT hPGCLCs, 1390G3-AGVT hPGCLCs, 585B1-AGDT hPGCLCs, 585B1-AGVT hPGCLCs, and 1383D6 hPGCLCs in a medium for differentiation induction to oogonia or prospermatogonia, and measuring the proliferation rate of fluorescent reporter/surface antigen marker-positive cells over time (magnification of the number of cells relative to day 0 as 1).

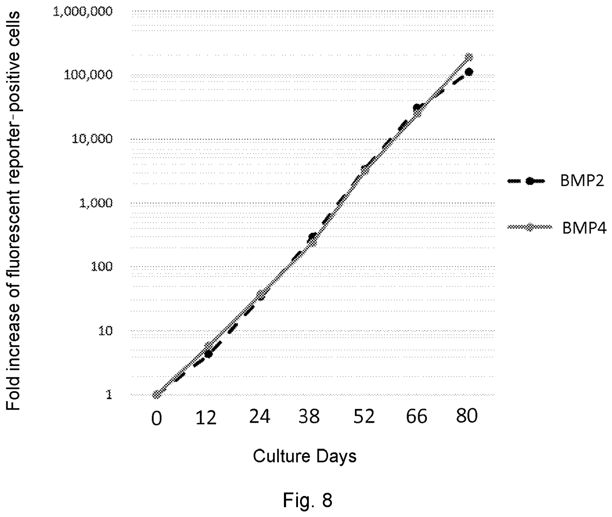

[Fig. 8] A diagram showing the results of culturing NCLCN-AGVT hPGCLCs under two conditions: adding 50 ng/ml BMP2 (BMP2) to the medium for differentiation induction to oogonia or prospermatogonia, or adding 50 ng/ml BMP4 instead of BMP2 (BMP4), and measuring the proliferation rate of fluorescent reporter-positive cells (magnification of the number of cells relative to day 0 as 1) at day 12, day 24, day 38, day 52, day 66, and day 80.

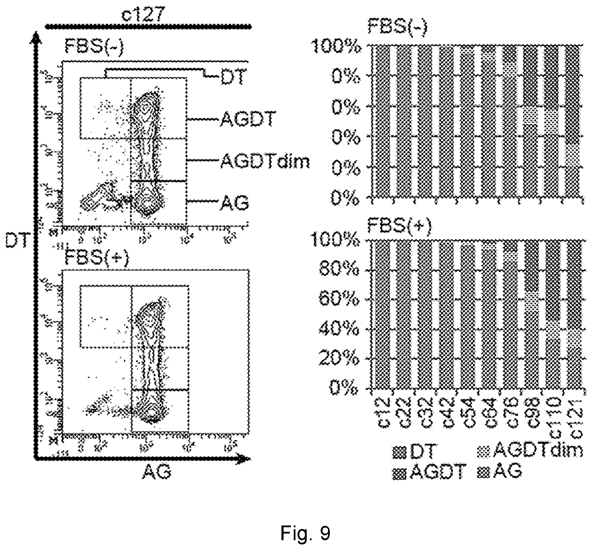

[Fig. 9] Graphs analyzing 585B1-AGDT hPGCLCs cultured in a medium for differentiation induction to oogonia or prospermatogonia from which fetal bovine serum (FBS) was removed (FBS(-)) by fluorescence intensity of DT (vertical axis) and AG (horizontal axis) on day 127 (c127) (Left figure, upper row) and graphs showing the ratios of AG+DT- cells (AG), AG+DT+ cells (AGDT), AG+DT dim positive (AGDT dim), and AG-DT+ cells (DT) from day 12 to day 121 (c12 to c121) (Right figure, upper row). As a control, the results of culturing 585B1-AGDT hPGCLCs in a medium for differentiation induction to oogonia or prospermatogonia without removing FBS (FBS(+)) are shown in the lower row of the left figure and the lower row of the right figure.

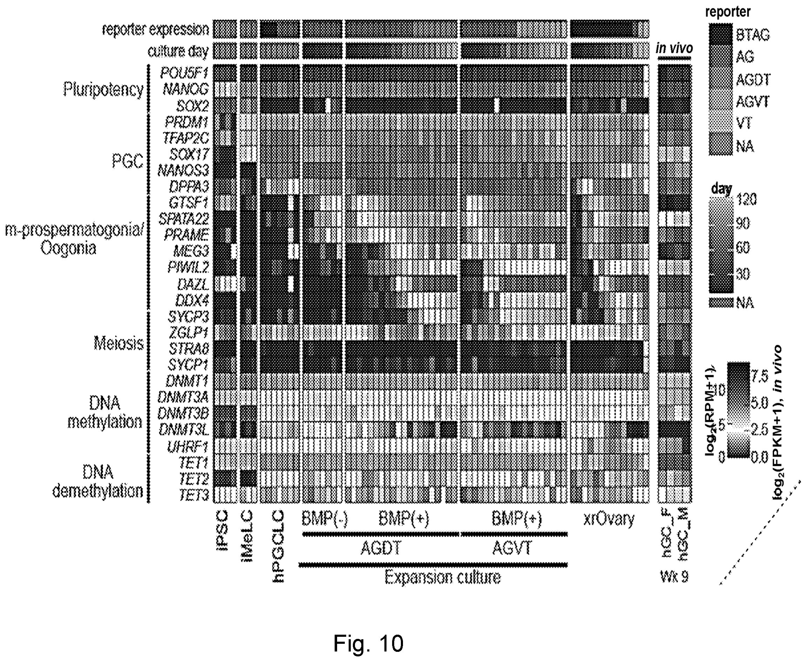

[Fig. 10] A diagram showing the comparison of RNA sequencing (RNA-seq) results of iPSCs, iMeLCs, hPGCLCs, 585B1-AGDT and NCLCN-AGVT cells cultured for various days in a medium for differentiation induction to oogonia or prospermatogonia showing reporter expressions of AG+DT- cells, AG+DT+ cells, or AG+VT- cells, AG+VT+ cells, AG-VT+ cells, respectively, and 585B1-AGDT cells cultured using a medium for differentiation induction to oogonia or prospermatogonia from which only BMP2 was excluded (BMP(-)), with known data of hPGCLC-induced cells in xrOvaries (xrOvary) and in vivo pre-mitotic spermatogonia/oogonia (hGC_M or hGC_F), showing the differences in expression levels of representative genes by a heatmap.

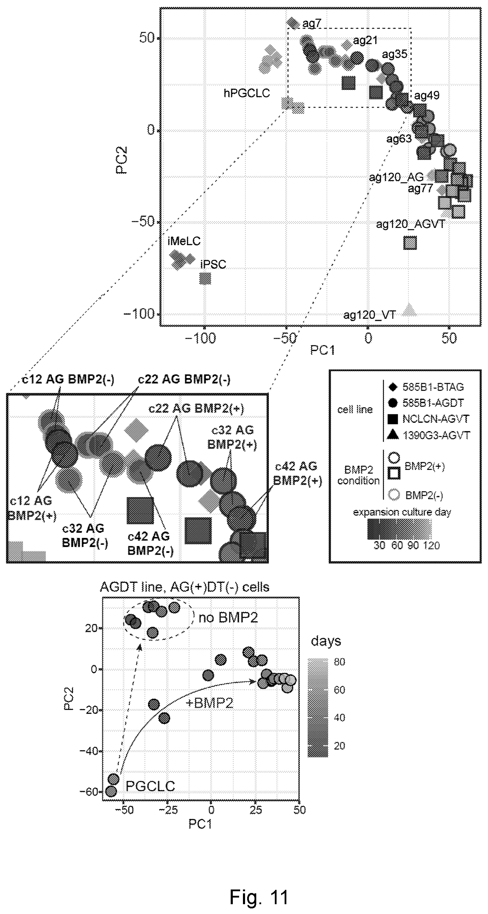

[Fig. 11] A diagram showing the results of principal component analysis (PCA) on gene expression data, comparing RNA sequencing results of iPSCs, iMeLCs, hPGCLCs, 585B1-AGDT and NCLCN-AGVT cells cultured for various days in a medium for differentiation induction to oogonia or prospermatogonia showing reporter expressions of AG+DT- cells, AG+DT+ cells, or AG+VT- cells, AG+VT+ cells, respectively, and 585B1-AGDT cells cultured using a medium for differentiation induction to oogonia or prospermatogonia from which only BMP2 was excluded (BMP(-)), with hPGCLC differentiation induction in xrOvaries. c[number] indicates cells on day [number] cultured in a medium for differentiation induction to oogonia or prospermatogonia, and ag[number] indicates cells on day [number] from the start of hPGCLC differentiation induction in xrOvaries.

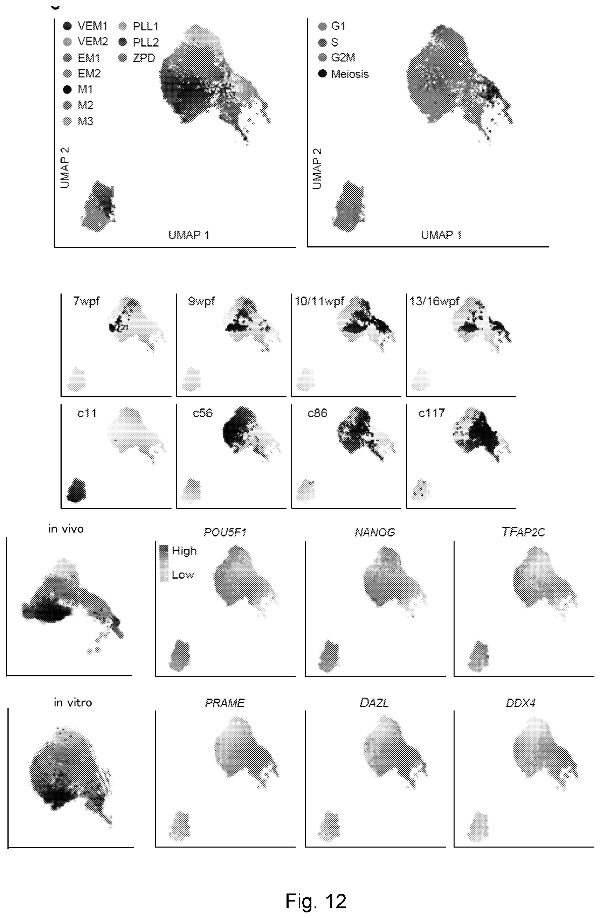

[Fig. 12] A diagram showing the results of comparative analysis of single-cell RNA-seq (scRNA-seq) analysis results of NCLCN-AGVT hPGCLCs cultured in a medium for differentiation induction to oogonia or prospermatogonia on culture day 11 (c11), day 56 (c56), day 86 (c86), and day 117 (c117), and data of in vivo oogonia and fetal eggs in meiotic prophase I at 7 to 16 wpf, by dimensionality reduction using Uniform manifold approximation and projection (UMAP) and Louvain clustering. Annotated into 10 clusters: very early mitosis (VEM) 1/2, early mitosis (EM) 1/2, mitosis (M) 1/2/3, pre-leptotene (PLL1), leptotene (PLL2), and zygotene/pachytene/diplotene (ZPD).

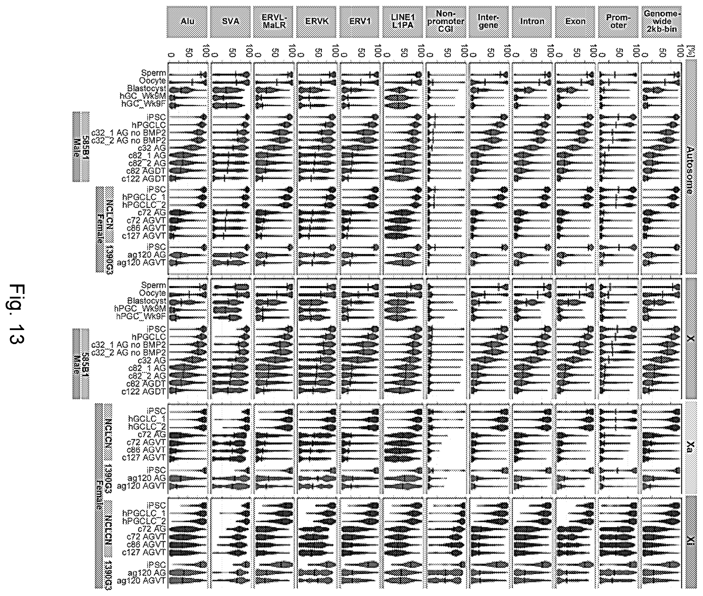

[Fig. 13] A graph showing violin plots of 5mC levels from enzymatic methyl sequencing (EM-seq) or whole genome bisulfite sequencing results of 585B1-AGDT or NCLCN-AGVT hiPSCs, 585B1-AGDT or NCLCN-AGVT hPGCLCs, AG+DT- cells on day 32 obtained by culturing 585B1-AGDT hPGCLCs in a medium for differentiation induction to oogonia or prospermatogonia from which only BMP2 was excluded (c32 AG no BMP2), AG+DT- cells on day 32 (Male: 585B1: c32 AG), AG+DT- cells on day 82 (Male: 585B1: c82 AG), AG+DT+ cells on day 82 (Male: 585B1: c82 AGDT), and AG+DT+ cells on day 122 (Male: 585B1: c122 AGDT) obtained by culturing 585B1-AGDT hPGCLCs in a medium for differentiation induction to oogonia or prospermatogonia (containing BMP2), AG+VT- cells on day 72 (Female: NCLCN: c72 AG), AG+VT+ cells on day 72 (Female: NCLCN: c72 AGVT), AG+VT+ cells on day 86 (Female: NCLCN: c86 AGVT), and AG+VT+ cells on day 127 (Female: NCLCN: c127 AGVT) obtained by culturing NCLCN-AGVT hPGCLCs in a medium for differentiation induction to oogonia or prospermatogonia (containing BMP2), and female 1390G3 strain hPGCLCs on day 120 of hPGCLC differentiation induction in xrOvaries (Female: 1390G3: ag120 AG and ag120 AGVT), and data of sperm (Sperm), oocytes (Oocyte), blastocysts (Blastocyst), and in vivo pre-mitotic spermatogonia/oogonia at week 9 (hGC_Wk9M or hGC_Wk9F).

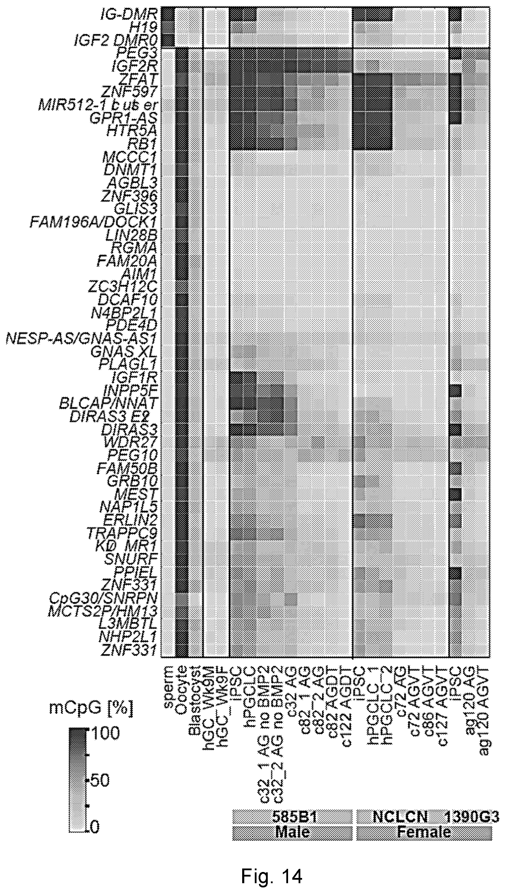

[Fig. 14] A diagram showing the 5mC levels of methylated regions (DMRs) of imprinted genes by a heatmap, from the enzymatic methyl sequencing (EM-seq) or whole genome bisulfite sequencing results of 585B1-AGDT or NCLCN-AGVT hiPSCs, 585B1-AGDT or NCLCN-AGVT hPGCLCs, AG+DT- cells on day 32 obtained by culturing 585B1-AGDT hPGCLCs in a medium for differentiation induction to oogonia or prospermatogonia from which only BMP2 was excluded (c32 AG no BMP2), AG+DT- cells on day 32 (Male: 585B1: c32 AG), AG+DT-cells on day 82 (Male: 585B1: c82 AG), AG+DT+ cells on day 82 (Male: 585B1: c82 AGDT), and AG+DT+ cells on day 122 (Male: 585B1: c122 AGDT) obtained by culturing 585B1-AGDT hPGCLCs in a medium for differentiation induction to oogonia or prospermatogonia (containing BMP2), AG+VT- cells on day 72 (Female: NCLCN: c72 AG), AG+VT+ cells on day 72 (Female: NCLCN: c72 AGVT), AG+VT+ cells on day 86 (Female: NCLCN: c86 AGVT), and AG+VT+ cells on day 127 (Female: NCLCN: c127 AGVT) obtained by culturing NCLCN-AGVT hPGCLCs in a medium for differentiation induction to oogonia or prospermatogonia (containing BMP2), and female 1390G3 strain hPGCLCs on day 120 of hPGCLC differentiation induction in xrOvaries (Female: 1390G3: ag120 AG and ag120 AGVT), and data of sperm (Sperm), oocytes (Oocyte), blastocysts (Blastocyst), and in vivo pre-mitotic spermatogonia/oogonia at week 9 (hGC_Wk9M or hGC_Wk9F).

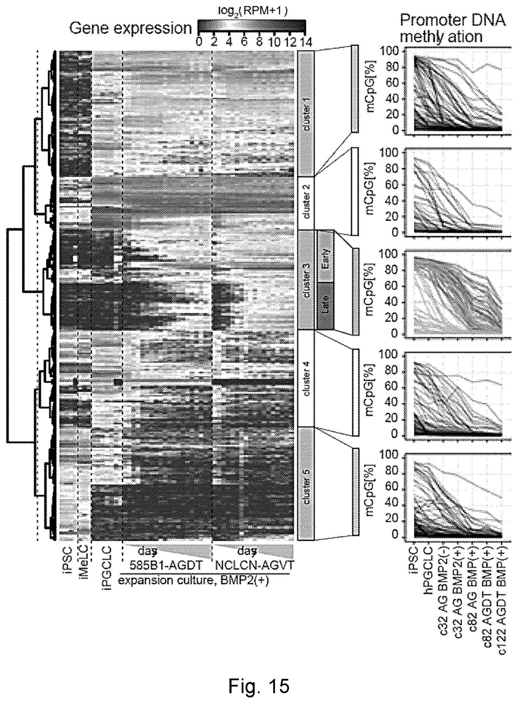

[Fig. 15] A diagram showing the results of unsupervised hierarchical cluster analysis (UHC) of gene expression dynamics from RNA-seq results for hiPSCs, hiMeLCs, hPGCLCs, and 585B1-AGDT and NCLCN-AGVT cells cultured for various days in a medium for differentiation induction to oogonia or prospermatogonia, where reporter expression is AG+DT+ cells or AG+VT+ cells, respectively (Left figure), and a graph showing the 5mC levels of the promoter regions of the respective genes (Right figure).

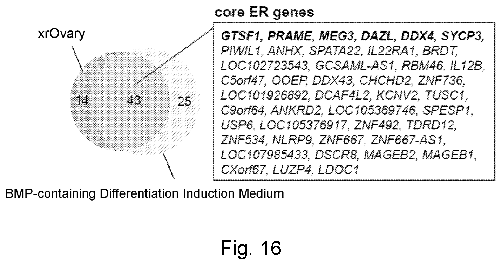

[Fig. 16] A schematic diagram listing overlapping gene groups as core ER genes from the epigenetic reprogramming activation gene (ER gene) group revealed by hPGCLC differentiation induction in xrOvaries and the ER gene group revealed by differentiation induction from hPGCLCs using a medium for differentiation induction to oogonia or prospermatogonia containing BMP2.

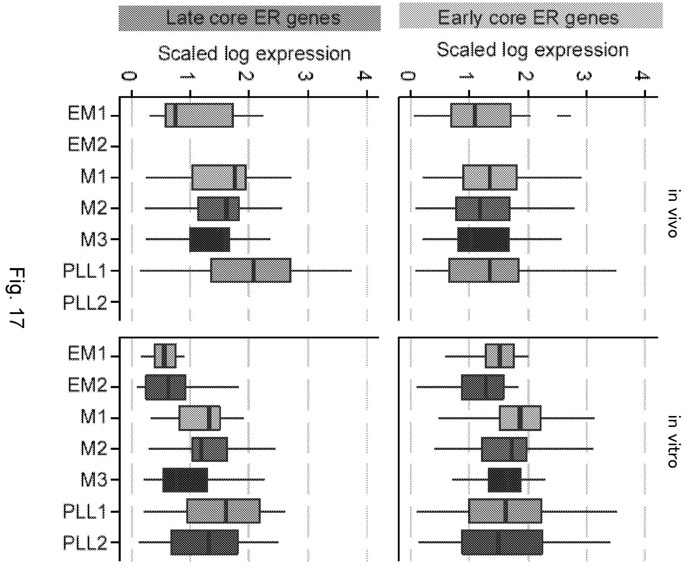

[Fig. 17] A diagram showing the expression of early core ER genes and late core ER genes by violin plots for data of 7 to 16 wpf oogonia and fetal eggs in meiotic prophase I annotated into 7 clusters of EM1/2, M1/2/3, PLL1, and PLL2 (in vivo), and for cells on culture day 11, day 56, day 86, and day 117 obtained by culturing NCLCN-AGVT hPGCLCs in a medium for differentiation induction to oogonia or prospermatogonia, annotated into 7 clusters of EM1/2, M1/2/3, PLL1, and PLL2 (in vitro).

### Embodiments for Carrying Out the Invention

The method for producing oogonia or prospermatogonia of the present invention comprises a step of culturing PGCLCs in a medium containing a BMP receptor type 2 signal activator to obtain oogonia or prospermatogonia.

### <PGCLCs>

PGCLCs that can be used in the method of the present invention may be any cells that are induced in vitro from pluripotent stem cells and have characteristics equivalent to PGCs, but are preferably cells positive for pluripotency marker genes POU5F1 (also called Oct3/4) and NANOG, and positive for PRDM1 (full name PR/SET domain 1, also called BLIMP1), TFAP2C (full name Transcription Factor AP-2 gamma, also called ERF1), and SOX17. Positivity for these markers can be confirmed by analyzing protein expression by immunostaining using antibodies or the like, or by analyzing gene expression by RT-PCR or the like.

PGCLCs may be human PGCLCs or PGCLCs of non-human mammals such as mice and rats, but humans are preferred.

PGCLCs can be obtained, for example, by inducing differentiation from pluripotent stem cells by the methods shown in any of the following.
WO 2012/020687
Cell, 146(4), 519-532 (2011)
WO 2019/107576
WO 2022/039279

Hereinafter, pluripotent stem cells that can be used as starting cells for PGCLCs will be described.

### <Pluripotent Stem Cells>

Pluripotent stem cells are stem cells that have pluripotency capable of differentiating into many cells existing in the living body and also have proliferative ability, and include any cells that are induced to differentiate into blastocyst-like structures. Pluripotent stem cells include, but are not particularly limited to, embryonic stem (ES) cells, induced pluripotent stem (iPS) cells, embryonic germ cells (EG cells), and the like. Preferred pluripotent stem cells are iPS cells and ES cells. The origin of the pluripotent stem cells is preferably mammalian, more preferably primate or rodent, and even more preferably human.

As ES cells, ES cells established by known methods can be used. Methods for establishing and maintaining ES cells are described, for example, in USP 5,843,780; Thomson JA, et al. (1995), Proc Natl. Acad. Sci. U S A. 92:7844-7848; Thomson JA, et al. (1998), Science. 282:1145-1147; H. Suemori et al. (2006), Biochem. Biophys. Res. Commun., 345:926-932; M. Ueno et al. (2006), Proc. Natl. Acad. Sci. USA, 103:9554-9559; H. Suemori et al. (2001), Dev. Dyn., 222:273-279; H. Kawasaki et al. (2002), Proc. Natl. Acad. Sci. USA, 99:1580-1585; Klimanskaya I, et al. (2006), Nature. 444:481-485, and the like. Human ES cell lines, for example, WA01 (H1) and WA09 (H9) are available from WiCell Research Institute, and KhES-1, KhES-2, and KhES-3 are available from the Institute for Frontier Medical Sciences, Kyoto University (Kyoto, Japan).

Embryonic germ cells are cells having pluripotency similar to ES cells, established from primordial germ cells in the fetal period, and can be established by culturing primordial germ cells in the presence of substances such as LIF, bFGF, and stem cell factor (Y. Matsui et al. (1992), Cell, 70:841-847; J.L. Resnick et al. (1992), Nature, 359:550-551).

Induced pluripotent stem (iPS) cells are artificial stem cells derived from somatic cells that can be produced by introducing specific reprogramming factors into somatic cells in the form of DNA or protein, and have characteristics substantially equivalent to ES cells, for example, differentiation pluripotency and proliferative ability by self-renewal (K. Takahashi and S. Yamanaka (2006) Cell, 126:663-676; K. Takahashi et al. (2007), Cell, 131:861-872; J. Yu et al. (2007), Science, 318:1917-1920; Nakagawa, M. et al. (2008), Nat. Biotechnol. 26:101-106; WO 2007/069666).

The reprogramming factors may be constituted by genes specifically expressed in ES cells, their gene products or non-coding RNAs, genes playing an important role in maintaining the undifferentiated state of ES cells, their gene products or non-coding RNAs, or low molecular weight compounds having equivalent functions. Numerous genes have been reported as genes included in reprogramming factors, and they are not particularly limited, but examples include Oct3/4, Sox2, Sox1, Sox3, Sox15, Sox17, KIf4, KIf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, or Glis1, etc., and these may be used alone or in combination.

The reprogramming factors may also include factors used for the purpose of enhancing the establishment efficiency of iPS cells, such as histone deacetylase (HDAC) inhibitors (e.g., low molecular weight inhibitors such as valproic acid (VPA), trichostatin A, sodium butyrate, MC 1293, M344, nucleic acid expression inhibitors such as siRNA and shRNA against HDAC, etc.), MEK inhibitors (e.g., PD184352, PD98059, U0126, SL327, and PD0325901), Glycogen synthase kinase-3 inhibitors (e.g., Bio and CHIR99021), DNA methyltransferase inhibitors (e.g., 5-azacytidine), histone methyltransferase inhibitors (e.g., low molecular weight inhibitors such as BIX-01294, nucleic acid expression inhibitors such as siRNA and shRNA against Suv39h1, Suv39h2, SetDB1, and G9a, etc.), L-channel calcium agonists (e.g., Bayk8644), butyric acid, TGFβ inhibitors or ALK5 inhibitors (e.g., LY364947, SB431542, 616453, and A-83-01), p53 inhibitors (e.g., siRNA and shRNA against p53), ARID3A inhibitors (e.g., siRNA and shRNA against ARID3A), miRNAs such as miR-291-3p, miR-294, miR-295, and mir-302, Wnt Signaling (e.g., soluble Wnt3a), neuropeptide Y, prostaglandins (e.g., prostaglandin E2 and prostaglandin J2), hTERT, SV40LT, UTF1, IRX6, GLIS1, PITX2, DMRT1, etc., and these can also be used as reprogramming factors.

iPS cells may be produced and used by introducing reprogramming factors into somatic cells as described above, or may be obtained from cell banks or the like and used.

### <PGCs>

In the method of the present invention, PGCs can also be used instead of PGCLCs.

PGCs are preferably cells positive for pluripotency marker genes POU5F1 (also called Oct3/4) and NANOG, and positive for PRDM1 (full name PR/SET domain 1, also called BLIMP1), TFAP2C (full name Transcription Factor AP-2 gamma, also called ERF1), and SOX17.

PGCs may be human PGCs or PGCs of non-human mammals such as mice and rats.

### PGCs can be isolated by any method known per se in the art (see, for example,

Patent Document 1). For example, they can be isolated from tissues of dead fetuses at about 5 to 9 weeks of gestation by techniques such as FACS using the expression of PGC-specific markers as an indicator.

In the case of mice, for example, they can be isolated from embryos at embryonic day (E) 9.5 to 11.5 by techniques such as FACS using the expression of PGC-specific markers as an indicator. Alternatively, PGCs similarly isolated from earlier mouse embryos can be cultured to a stage corresponding to migratory PGCs and used. Mammals other than mice can also be prepared in the same manner from embryos of fetal age corresponding to the fetal age of the mouse, respectively.

### <Oogonia>

Oogonia are mitotic undifferentiated egg cells (cells that have not started meiosis) located between primordial germ cells and oocytes, and are cells that are positive for, in addition to the above PGCLC marker genes, one or more of the following, preferably 10 or more of the following, and more preferably all of the following markers. It is preferable that at least DAZL (Deleted In Azoospermia Like) and DDX4 (DEAD-Box Helicase 4) are positive.
GTSF1 (Human Gene ID: 121355, Mouse Gene ID: 74174),
PRAME (Human Gene ID: 23532),
MEG3 (Human Gene ID: 55384, Mouse Gene ID: 17263),
DAZL (Human Gene ID: 1618, Mouse Gene ID: 13164),
DDX4 (Human Gene ID: 54514, Mouse Gene ID: 13206),
SYCP3 (Human Gene ID: 50511, Mouse Gene ID: 20962),
PIWIL1 (Human Gene ID: 9271, Mouse Gene ID: 57749),
ANHX (Human Gene ID: 647589),
SPATA22 (Human Gene ID: 84690, Mouse Gene ID: 380709), IL22RA1,
BRDT (Human Gene ID: 676, Mouse Gene ID: 114642),
LOC102723543 (Human Gene ID: 102723543),
GCSAML-AS1 (Human Gene ID: 148824),
RBM46 (Human Gene ID: 166863, Mouse Gene ID: 633285),
IL12B (Human Gene ID: 3593, Mouse Gene ID: 16160),
C5orf47 (Human Gene ID: 133491),
OOEP (Human Gene ID: 441161, Mouse Gene ID: 7968),
DDX43 (Human Gene ID: 55510, Mouse Gene ID: 100048658),
CHCHD2 (Human Gene ID: 51142, Mouse Gene ID: 14004),
ZNF736 (Human Gene ID: 728927),
LOC101926892 (Human Gene ID: 101926892),
DCAF4L2 (Human Gene ID: 138009)
KCNV2 (Human Gene ID: 169522, Mouse Gene ID: 240595)
TUSC1 (Human Gene ID: 286319, Mouse Gene ID: 69136)
C9orf64 (Human Gene ID: 84267, Mouse Gene ID: 70153)
ANKRD2 (Human Gene ID: 26287, Mouse Gene ID: 56642)
LOC1053697 (Human Gene ID: 105369746)
SPESP1 (Human Gene ID: 246777, Mouse Gene ID: 66712)
USP6 (Human Gene ID: 9098)
LOC105376917 (Human Gene ID: 105376917)
ZNF492 (Human Gene ID: 57615, Mouse Gene ID: 238722)
TDRD12 (Human Gene ID: 91646, Mouse Gene ID: 71981)
ZNF534 (Human Gene ID: 147658)
NLRP9 (Human Gene ID: 338321, Mouse Gene ID: 330490)
ZNF667 (Human Gene ID: 63934)
ZNF667-AS1 (Human Gene ID: 100128252)
LOC107985433 (Human Gene61 ID: 107985433)
DSCR8 (Human Gene ID: 84677)
MAGEB2 (Human Gene ID: 4113)
MAGEB1 (Human Gene ID: 4112)
CXorf67 (Human Gen72e ID: 340602)
LUZP4 (Human Gene ID: 51213),
LDOC1 75(Human Gene ID: 23641, Mouse Gene ID: 434784).

### <Prospermatogonia>

Prospermatogonia are mitotic cells (cells that have not started meiosis) located between primordial germ cells and spermatogonia, and are cells that are positive for, in addition to the PGCLC marker genes, one or more of the following, preferably 2 or more of the following, more preferably 10 or more of the following, and even more preferably all of the following markers. It is preferable that at least DAZL and DDX4 are positive.
GTSF1, PRAME, MEG3, DAZL, DDX4, SYCP3, PIWIL1, ANHX, SPATA22, IL22RA1, BRDT, LOC102723543, GCSAML-AS1, RBM46, IL12B, C5orf47, OOEP, DDX43, CHCHD2, ZNF736, LOC101926892, DCAF4L2, KCNV2, TUSC1, C9orf64, ANKRD2, LOC105369746, SPESP1, USP6, LOC105376917, ZNF492, TDRD12, ZNF534, NLRP9, ZNF667, ZNF667-AS1, LOC107985433, DSCR8, MAGEB2, MAGEB1, CXorf67, LUZP4, LDOC1.

In addition, oogonia and prospermatogonia preferably have reduced genome-wide DNA methylation levels. Specifically, for example, the genome-wide DNA methylation level is preferably reduced to 50% or less, 40% or less, 30% or less, 25% or less, 20% or less, 15% or less, or 10% or less. More specifically, it is preferably reduced to the same level (about 10% of the total) or lower than that of germ cells of a 7-9 week old human fetus.

To obtain oogonia from PGCLCs or PGCs, cells with XX sex chromosomes may be used as PGCLCs or PGCs, and to obtain prospermatogonia from PGCLCs or PGCs, cells with XY sex chromosomes may be used as PGCLCs or PGCs.

### <Culture Method>

PGCLCs or PGCs are cultured in a medium containing a BMP receptor type 2 signal activator.

The BMP receptor type 2 signal activator is not particularly limited as long as it is a substance capable of binding to BMPR-type 2 and activating the BMPR-type 2 signaling pathway, but BMP2, BMP4, BMP5, BMP6, BMP7, BMP8A, BMP8B, BMP9, and BMP10 are preferred, and BMP2, BMP4, and BMP7 are more preferred.

Small molecule compounds activating the BMPR-type 2 signaling pathway can also be used. For example, a compound called sb4 has been reported as a small molecule compound that substitutes for signaling of BMP2, BMP4, and BMP7 in activating the BMPR-type 2 signaling pathway (Bradford, et al., Journal of Biological Chemistry, 2019, doi.org/10.1074/jbc.RA118.006817).

The BMPR-type 2 signaling pathway includes a pathway that phosphorylates intracellular SMAD1/5/8 and causes downstream gene expression (Mousakas and Heldin, Development, 2009, doi.org/10.1242/dev.030338).

The concentration of the BMP receptor type 2 signal activator is not particularly limited as long as it can induce differentiation of oogonia or prospermatogonia from PGCLCs or PGCs. For example, when BMP2 is used as the BMP receptor type 2 signal activator, the concentration of BMP2 may be, for example, 10 ng/ml or more, preferably 20 ng/ml or more, and more preferably 25 ng/ml or more. Also, the concentration of BMP2 may be, for example, 500 ng/ml or less, preferably 250 ng/ml or less, and more preferably 200 ng/ml or less. Alternatively, the concentration of BMP2 may be any non-contradictory combination of these. Other BMPs can be in similar concentration ranges.

The Wnt signal inhibitor can suppress dedifferentiation of PGCLCs or PGCs. Therefore, the medium may further contain a Wnt signal inhibitor. Thereby, dedifferentiation of PGCLCs or PGCs is suppressed, and differentiation induction efficiency from PGCLCs or PGCs to oogonia or prospermatogonia can be improved.

That is, in one embodiment, the method for producing oogonia or prospermatogonia comprises a step of culturing primordial germ cell-like cells or primordial germ cells in a medium containing a BMP receptor type 2 signal activator and a Wnt signal inhibitor to obtain oogonia or prospermatogonia. The Wnt signal inhibitor is not particularly limited, and examples thereof include substances that promote degradation of beta-catenin and/or inhibit nuclear translocation. More specifically, examples include substances that stabilize axin. Further specifically, examples include substances that inhibit tankyrase. Examples of substances that inhibit tankyrase include IWR1 (also called IWR1-endo), XAV939, IWR2, JW55, JW74, G007-LK, NVP-TNKS656, WIKI4, MN-64, TC-E 5001, and AZ6102.

Among these, IWR1 or XAV939 may be particularly preferred.

These Wnt signal inhibitors can be obtained, for example, from SIGMA Aldrich.

Other examples of Wnt signal inhibitors include PNU-74654, KY02111, etc., which inhibit beta-catenin.

Other examples of Wnt signal inhibitors include substances that inhibit Wnt secretion (e.g., Wnt-C59, IWP2), and the like.

Another example of a Wnt signal inhibitor is Wnt-C59.

Other examples of Wnt signal inhibitors include proteins such as IGFBP4 and DKK1, antisense nucleic acids that suppress the expression or function of Wnt and proteins constituting the Wnt signaling pathway, RNA interference inducing nucleic acids (e.g., siRNA), competitive peptides, antagonist peptides, inhibitory antibodies, antibody-ScFV fragments, dominant negative mutants, and expression vectors thereof.

These Wnt signal inhibitors may be used alone or in combination of two or more.

The concentration of the Wnt signal inhibitor may be any concentration that exerts a Wnt signaling inhibitory effect, and can be appropriately set depending on the type of Wnt signal inhibitor. For example, it may be about 0.1 µM or more, preferably about 0.5 µM or more, and more preferably about 1 µM or more. Also, the concentration of the Wnt signal inhibitor may be, for example, about 10 µM or less, preferably about 5 µM or less, and more preferably about 2.5 µM or less. Alternatively, the concentration of the Wnt signal inhibitor may be any non-contradictory combination of these.

The Wnt signal inhibitor may be added throughout the entire period of the maintenance and proliferation culture of the present invention, or may be added during any period (which may be continuous or intermittent) during the maintenance culture period. The lower limit of the addition period is also not particularly limited, and examples include 1 day or more, 2 days or more, 3 days or more, 4 days or more, 5 days or more, 10 days or more, 15 days or more, 20 days or more, and the like.

In addition to the BMP receptor type 2 signal activator, the medium may further contain one or more of stem cell factor (SCF), cAMP enhancer, and basic FGF (bFGF). Examples of the cAMP enhancer include forskolin and the like. That is, in one embodiment, the method for producing oogonia or prospermatogonia comprises a step of culturing primordial germ cell-like cells or primordial germ cells in a medium containing a BMP receptor type 2 signal activator and one or more of stem cell factor (SCF), cAMP enhancer, and basic FGF (bFGF) to obtain oogonia or prospermatogonia. In another embodiment, the method for producing oogonia or prospermatogonia comprises a step of culturing primordial germ cell-like cells or primordial germ cells in a medium containing a BMP receptor type 2 signal activator, a Wnt signal inhibitor, and one or more of stem cell factor (SCF), cAMP enhancer, and basic FGF (bFGF) to obtain oogonia or prospermatogonia.

The concentration of SCF may be, for example, 10 ng/ml or more, preferably 20 ng/ml or more, more preferably 25 ng/ml or more, and may be 500 ng/ml or less, preferably 250 ng/ml or less, more preferably 200 ng/ml or less, or any non-contradictory combination thereof.

The concentration of the cAMP enhancer (e.g., forskolin) may be, for example, 1 µM or more, preferably 5 µM or more, and may be 100 µM or less, preferably 50 µM or less, or any non-contradictory combination thereof.

The concentration of bFGF may be, for example, 1 ng/ml or more, preferably 5 ng/ml or more, more preferably 10 ng/ml or more, and may be 200 ng/ml or less, preferably 100 ng/ml or less, more preferably 50 ng/ml or less, or any non-contradictory combination thereof.

As the medium, a medium generally used for culturing animal cells can be used as a basal medium, and can be prepared by adding the above BMP receptor type 2 signal activator and, if necessary, a Wnt signal inhibitor, SCF, cAMP enhancer, bFGF, etc.

Examples of the basal medium include media that can be used for culturing animal cells, such as BME medium, BGJb medium, CMRL 1066 medium, Glasgow MEM (GMEM) medium, Improved MEM Zinc Option medium, IMDM medium, Medium 199 medium, Eagle MEM medium, alpha MEM medium, Advanced RPMI 1640 medium, DMEM medium, advanced MEM medium, StemPro34 medium, Ham's medium, RPMI 1640 medium, Fischer's medium, Neurobasal medium, IVF medium, or mixed media thereof. Among these, Advanced RPMI 1640 and DMEM are preferred.

The basal medium may be a serum-containing medium or a serum-free medium. Serum-free medium (SFM) means a medium that does not contain any untreated or unpurified serum. Thus, SFM may include, but is not limited to, media containing purified blood-derived components or animal tissue-derived components (such as growth factors). SFM may or may not contain any serum replacements. Examples of serum replacements may include substances appropriately containing albumin (e.g., lipid-rich albumin, albumin substitutes such as recombinant albumin, plant starch, dextran, protein hydrolysates, etc.), transferrin (or other iron transporters), fatty acids, insulin, collagen precursors, trace elements, 2-mercaptoethanol, 3'-thioglycerol, or equivalents thereof. Such serum replacements can be prepared, for example, by the method described in WO 98/30679. In addition, commercially available ones can be used for greater simplicity. Examples of such commercially available substances include Knockout (trademark) Serum Replacement (KSR), Chemically-defined Lipid concentrated, and Glutamax (Invitrogen).

The basal medium may contain other additives. Additives are not particularly limited, but examples include growth factors (e.g., insulin etc.), polyamines (e.g., putrescine etc.), minerals (e.g., sodium selenate etc.), saccharides (e.g., glucose etc.), organic acids (e.g., pyruvate, lactic acid etc.), amino acids (e.g., non-essential amino acids (NEAA), L-glutamine etc.), reducing agents (e.g., 2-mercaptoethanol etc.), vitamins (e.g., ascorbic acid, d-biotin etc.), steroids (e.g., [beta]-estradiol, progesterone etc.), antibiotics (e.g., streptomycin, penicillin, gentamicin etc.), buffers (e.g., HEPES etc.), and the like. Each additive is preferably included in a concentration range known per se.

Note that the medium may be a medium that does not contain retinoic acid. That is, in one embodiment, the method for producing oogonia or prospermatogonia comprises a step of culturing primordial germ cell-like cells or primordial germ cells in a medium containing a BMP receptor type 2 signal activator and not containing retinoic acid to obtain oogonia or prospermatogonia.

In another embodiment, the method for producing oogonia or prospermatogonia comprises a step of culturing primordial germ cell-like cells or primordial germ cells in a medium containing a BMP receptor type 2 signal activator and a Wnt signal inhibitor, and not containing retinoic acid, to obtain oogonia or prospermatogonia.

In another embodiment, the method for producing oogonia or prospermatogonia comprises a step of culturing primordial germ cell-like cells or primordial germ cells in a medium containing a BMP receptor type 2 signal activator and one or more of stem cell factor (SCF), cAMP enhancer, and basic FGF (bFGF), and not containing retinoic acid, to obtain oogonia or prospermatogonia.

In another embodiment, the method for producing oogonia or prospermatogonia comprises a step of culturing primordial germ cell-like cells or primordial germ cells in a medium containing a BMP receptor type 2 signal activator, a Wnt signal inhibitor, and one or more of stem cell factor (SCF), cAMP enhancer, and basic FGF (bFGF), and not containing retinoic acid, to obtain oogonia or prospermatogonia.

In addition, a ROCK (Rho kinase) inhibitor can be added to the medium to suppress cell death and efficiently culture, passage, proliferate cells, and/or differentiate. As the ROCK inhibitor, known ones can be appropriately used, and examples include Y-27632 and the like. The concentration of the ROCK inhibitor is, for example, 1 to 50 µM, preferably 5 to 20 µM. It is preferable to add the ROCK inhibitor to the medium for at least 3 days after the start of cell culture or passage.

Culturing may be performed by either adherent culture or non-adherent culture, and is not particularly limited, but is preferably performed by adherent culture. The concentration of PGCLCs or PGCs seeded at the start of culture is not particularly limited, but they can be seeded at a cell density of, for example, about 1 x 10^5 to 1 x 10^6 cells/mL, preferably about 2 x 10^5 to 5 x 10^5 cells/mL. The temperature and CO₂ concentration of the culture may be the same conditions as normal cell culture, for example, culturing can be performed at about 30 to about 40°C (preferably about 37°C) in an atmosphere of 1 to 10% CO₂ / 99 to 90% air. The culture period is not particularly limited as long as it is a period during which oogonia or prospermatogonia can be induced to differentiate from PGCLCs or PGCs, but may be, for example, 10 days or more, 20 days or more, 30 days or more, 40 days or more, 50 days or more, 60 days or more, 80 days or more, 100 days or more, or 120 days or more. Since oogonia or prospermatogonia can be maintained and cultured for a long period of time, there is no particular upper limit to the culture period, but for example, the upper limit of the culture period may be 300 days.

The culture vessel used is not particularly limited, but examples may include flasks, tissue culture flasks, dishes, petri dishes, tissue culture dishes, multi-dishes, microplates, microwell plates, multiplates, multiwell plates, microslides, chamber slides, petri dishes, tubes, trays, culture bags, and roller bottles. The culture vessel may be cell-adhesive. The cell-adhesive culture vessel may be coated with any cell adhesion substrate such as extracellular matrix (ECM) for the purpose of improving adhesion to cells on the surface of the culture vessel. Examples of cell adhesion substrates include collagen, gelatin, poly-L-lysine, poly-D-lysine, poly-L-ornithine, laminin, and fibronectin, and mixtures thereof, such as Matrigel, and lysed cell membrane preparations (Klimanskaya I et al 2005. Lancet 365: p1636-1641).

Culturing may be performed in the presence or absence of feeder cells. Feeder cells are not particularly limited, but examples include fibroblasts (mouse embryonic fibroblasts, mouse fibroblast lines STO, m220-5, etc.). The feeder cells are preferably inactivated by a method known per se, for example, treatment with radiation (gamma rays etc.), anticancer agents (mitomycin C etc.), or the like.

In the method of the present invention described above, oogonia or prospermatogonia can be obtained from PGCLCs even without providing an external environment that promotes differentiation induction into oogonia or prospermatogonia using xenogeneic ovarian cells or testicular cells, or ovarian cells or testicular cells derived from other individuals. From the viewpoint of obtaining oogonia or prospermatogonia as a pure cell population, the method of the present invention is preferably performed without including a step of providing an external environment that promotes differentiation induction into oogonia or prospermatogonia using xenogeneic ovarian cells or testicular cells, or ovarian cells or testicular cells derived from other individuals.

In the method of the present invention, oogonia or prospermatogonia can be differentiated and induced with high efficiency.

That is, by the method of the present invention, a cell population containing a high content of oogonia or prospermatogonia, for example, 80% or more, preferably 90% or more, can be obtained.

Therefore, one embodiment of the present invention relates to a cell population containing 80% or more, preferably 90% or more, of oogonia or prospermatogonia.

In order to increase the content of oogonia or prospermatogonia, oogonia or prospermatogonia may be selected using markers such as DDX4 and DAZL described above.

As described above, the "pure cell population of oogonia or prospermatogonia" obtained by one embodiment of the method of the present invention is preferably derived from a specific single individual, in other words, has a single genotype, from the viewpoint of being able to desirably control biological parentage or being able to identify biological parentage when applied to reproductive medicine or the like. Any conventionally known method can be adopted as a means for verifying that the genotype is single.

Further, in one embodiment of the method of the present invention, when the number of primordial germ cell-like cells or primordial germ cells is used as a reference starting point, it becomes possible to amplify the number of finally obtained oogonia or prospermatogonia up to 1 billion times from the reference cell number mentioned above.

### Examples

Hereinafter, the present invention will be described more specifically with reference to examples, but the present invention is not limited to the following embodiments.

### <Materials and Methods>

### Human iPS Cells (hiPSCs)

Cells used in all examples herein were maintained in a humidified incubator at 37°C, 5% CO₂. As hiPSCs, male hiPSCs (i.e., sex chromosomes are XY) 585B1 strain or 1383D6 strain, and female hiPSCs (i.e., sex chromosomes are XX) NCLCN strain or 1390G3 strain were used. hiPSCs were cultured on plates coated with Laminin-511 E8 fragment (iMATRIX-511; Nippi, 892014) using StemFit AK02N (Ajinomoto) or Stem Fit AK03N (Ajinomoto) as a medium. Passage of hiPSCs was performed by first dissociating hiPSC colonies into single cells using a 1:1 mixture of Tryple select (Gibco, 12563-011) and 0.5 mM EDTA (Nacalai Tesque, 06894-14) containing PBS(-), then seeding the dissociated cells in a medium containing 10 µM ROCK inhibitor (Y-27632; TOCRIS, 1254), and replacing with fresh medium not containing Y-27632 the next day.

### Reporter hiPSC Lines

TFAP2C-p2A-eGFP reporter (AG) and DAZL-p2A-tdTomato reporter (DT) were introduced into the 585B1 strain of hiPSCs, and TFAP2C-p2A-eGFP reporter (AG) and DDX4 (human VASA homolog)-p2A-tdTomato reporter (VT) were introduced into the NCLCN strain of hiPSCs, respectively. The structures of TFAP2C-p2A-eGFP, DAZL-p2A-tdTomato, and DDX4-p2A-tdTomato are as shown in Figure 1.

For introduction of TFAP2C-p2A-eGFP, DAZL-p2A-tdTomato, and DDX4-p2A-tdTomato reporters into hiPSCs, the stop codon of the target gene was replaced with p2A-eGFP or p2A-tdTomato by homologous recombination using TALENs.

Introduction of the TFAP2C-p2A-eGFP reporter is as described in "Robust In Vitro Induction of Human Germ Cell Fate from Pluripotent Stem Cells. Cell Stem Cell. 17, 178-194. (2015)."

Introduction of DAZL-p2A-tdTomato and DDX4-p2A-tdTomato reporters was performed as follows.

First, a homologous region around the stop codon of the human DAZL or human DDX4 gene was cloned into a pCR2.1 vector (pCR2.1-DAZL or pCR2.1-DDX4) (using TOPO TA Cloning; Thermo Fisher Scientific, 450641). Next, a p2A-tdTomato sequence linked to a PGK promoter-driven neomycin resistance gene flanked by loxP sites was subcloned into each of the pCR2.1-DAZL vector and pCR2.1-DDX4 vector using the GeneArt seamless cloning and assembly kit (Thermo Fisher Scientific, A13288). Finally, the MC1 promoter-driven DT-A gene was isolated by Xbal/Apal or Xhol/Apal restriction enzymes and subcloned downstream of the homologous region by enzymatic ligation.

To construct TALEN constructs targeting the human DAZL or human DDX4 locus, the GoldenGate TALEN and TAL Effector kit (Addgene, #1000000016) was used. The amino acid sequences of the variable di-residue repeats of each TALEN were designed as follows:
DAZL 5' end [NG NG NN NI NG HD HD NG HD HD NG NN NN HD NG NG NI NG HD];
DAZL 3' end [NI NG NG HD NI NI NI NI HD HD NI NN HD NI NI HD NG NG];
DDX4 5' end [HD HD HD NI NI NG HD HD NI NN NG NI NN NI NG NN NI NG];
DDX4 3' end [NN NI NI NN NN NI NG NN NG NG NG NG NN NN HD NG].

5 µg of donor vector and 2.5 µg of TALEN plasmid were introduced into 1.0 x 10^6 hiPSCs (585B1 or NCLCN with TFAP2C-p2A-eGFP already introduced) by electroporation using a NEPA21 type II electroporator (Nepagene). 150 µg/ml G418 was added 2-4 days after electroporation, and 0.2 µg/ml puromycin was added 4 days after electroporation to remove off-target cells. Approximately 2 weeks after electroporation, single colonies of hiPSCs were manually isolated using the tip of a pipette tip. The zygosity of the target locus and random integration in the isolated clones were evaluated by PCR using genomic DNA samples. Isolated clones having the reporter gene knocked-in heterozygously were then transfected with a Cre recombinase expression plasmid. Finally, the removal of the drug resistance gene flanked by loxP in the isolated clones was confirmed by PCR using genomic DNA samples. Thus, 585B1-TFAP2C-p2A-eGFP/DAZL-p2A-tdTomato (585B1-AGDT) and NCLCN-TFAP2C-p2A-eGFP/DDX4-p2A-tdTomato (NCLCN-AGVT) hiPSC lines, in which the DAZL-p2A-tdTomato reporter or DDX4-p2A-tdTomato reporter was introduced into 585B1 or NCLCN with TFAP2C-p2A-eGFP already introduced, respectively, were produced.

Karyotypes of 585B1-AGDT and NCLCN-AGVT were confirmed to be normal (46+XY or 46+XX, respectively) by G-banding analysis performed by Nihon Gene Research Laboratories Inc. (Miyagi Prefecture, Japan).

Differentiation induction to Human Primordial Germ Cell-Like Cells (hPGCLCs) hiPSCs were first induced to differentiate into incipient mesoderm-like cells (iMeLCs), and then induced to differentiate into hPGCLCs.

Differentiation induction to iMeLCs was performed by the following method. 1.0 to 2.0 x 10^5 cells/well of hiPSCs were seeded on fibronectin (Millipore, FC010)-coated 12-well plates in GK15 medium (GMEM (Gibco, 11710-035) medium containing 15% Knockout Serum Replacement (KSR) (Gibco, 10828-028), 1% MEM Non-Essential Amino Acids Solution (NEAA) (Gibco, 11140-050), 1% Penicillin & Streptomycin (Gibco, 15140-122), 2 mM L-Glutamine, 2 mM Sodium Pyruvate (Gibco, 11360-070), and 0.1 mM 2-Mercaptoethanol (Gibco, 21985-023)) supplemented with 3 µM CHIR99021 (TOCRIS, 4423), 50 ng/ml Activin A (PeproTech, AF-120-14), and 10 µM Y-27632. The induction period was 44 hours for 585B1-AGDT hiPSCs and 54 hours for NCLCN-AGVT hiPSCs.

Differentiation induction to hPGCLCs is as follows.

iMeLCs induced to differentiate as described above were seeded at 3.0 to 6.0 x 10^5 cells/well on v-bottom 96-well plates (Greiner, 651970) in GK15 medium supplemented with 200 ng/ml BMP4 (R&D SYSTEMS, 314-BP), 100 ng/ml SCF (R&D SYSTEMS, 255-SC), 10 ng/ml LIF (Merck Millipore, LIF1010), 50 ng/ml EGF (PeproTech, AF-100-15), and 10 µM Y-27632. Six days or eight days after the start of hPGCLC induction, aggregates of iMeLCs were subjected to FACS sorting to isolate hPGCLCs.

### Differentiation Induction to Oogonia or Prospermatogonia

hPGCLCs were cultured under the following conditions to induce differentiation to oogonia or prospermatogonia.

As a medium for differentiation induction to oogonia or prospermatogonia, a medium obtained by adding 15% KSR, 1x GlutaMAX (Gibco, 35050061), 1% Penicillin & Streptomycin, 0.1 mM 2-Mercaptoethanol, 2.5% Fetal Bovine Serum (FBS) (NICHIREI, 175012), 100 ng/ml hSCF (R&D Systems, 255-SC), 10 µM Forskolin (SIGMA, F3917), 20 ng/ml hbFGF (Wako, 064-04541), 1.5 µM IWR1 (SIGMA, I0161), and BMP2 (R&D SYSTEMS, 355-BM) to advanced RPMI (Gibco, 12633-012) was used. The amount of BMP2 added was 25 to 50 ng/ml from the seeding of hPGCLCs to the first passage, and 100 ng/ml after the first passage. 10 µM Y27632 was added only when cells were plated at the time of passage. At the start of culture, the first seeding of hPGCLCs was performed by sorting using a flow cytometer onto mitomycin C-treated m220-5 feeder cells, or by sorting into FACS buffer (0.1% BSA-PBS(-) containing 10 µM Y27632), collecting hPGCLCs by centrifugation at 200 g for 7 minutes, and seeding them.

### Passage of cultures was performed by the following procedure.

### 1. Preparation of culture plates and m220-5 feeder cells

1.1. A 0.1% gelatin solution was dispensed into flat-bottom cell culture plates (Falcon, 353072, etc.) and left to stand at room temperature for 1 hour or more for coating.

1.2. m220-5 feeder cells treated with 4 µg/ml mitomycin (MMC) for 2 hours were seeded on gelatin-coated cell culture plates. Usually, 6.25 x 10^4 cells were seeded in one well of a 96-well plate together with 0.125 ml of DMEM medium containing 10% FBS, 1% penicillin, streptomycin, and 1x GlutaMAX.

1.3. m220-5 feeder cells were cultured in a humidified incubator kept at 37°C, 5% CO₂ until use.

### 2. Cell harvesting and replating

2.1. Cultured cells were washed once with PBS(-).

2.2. Cultured cells were treated with a 1:4 mixture of 0.5% trypsin-EDTA and PBS(-) at 37°C for 5 minutes. Alternatively, cultured cells were dissociated using Accumax (AM105, Innovative Cell Technologies).

2.3. Advanced RPMI containing 10% FBS, 0.1 mg/mL DNase I (Sigma, DN25), and 10 µM Y27632 was added to neutralize the trypsin solution. Alternatively, the above-mentioned medium for differentiation induction to oogonia or prospermatogonia was used for neutralization.

2.4. Pipetting of the cell suspension was repeated to dissociate into single cells. 2.5. The medium of m220-5 feeder cells seeded in 1.3 was replaced with a medium for differentiation induction to oogonia or prospermatogonia supplemented with 10 µM Y27632, and the required amount of cell suspension was added. Usually, the recovered cell suspension was passaged at a dilution ratio of about 1:5.

2.6. Cells were cultured in a humidified incubator kept at 37°C, 5% CO₂.

### 3. Maintenance of proliferation culture

3.1. The medium was replaced with fresh medium on day 2 after seeding.

3.2. Half of the medium was replaced with fresh medium every other day until the next passage around day 10 after cell plating.

In addition, a control group "no BMP2" was also implemented in which hPGCLCs were cultured in the same procedure using the above-mentioned medium for differentiation induction to oogonia or prospermatogonia from which only BMP2 was excluded.

### Fluorescence Activated Cell Sorting (FACS)

Details of the method for isolating hPGCLCs from aggregates of iMeLCs by FACS sorting are as described in "Long-term expansion with germline potential of human primordial germ cell-like cells in vitro. EMBO J in press. (2020)." and are briefly described below.

Aggregates of iMeLCs were treated with a 1:1 mixture of PBS(-) and 0.5% trypsin-EDTA (Gibco, 15400-054). After neutralization with DMEM (Gibco, 10313-021) containing 10% FBS, 1% penicillin & streptomycin, 2 mM L-glutamine, 10 µM Y-27632, and 0.1 mg/mL DNase I, the cell suspension was centrifuged at 200 g for 5 minutes. Next, cells were resuspended in FACS buffer and sorted using FACS Aria III. hPGCLCs were selected as AG+ cells on day 6 or day 8 from hPGCLC induction.

### cDNAAmplification and qPCR

Total RNA was extracted from cells using the NucleoSpin RNA XS kit (MACHEREY-NAGEL, U0902A) according to the instructions. 1 ng of extracted RNA was used for reverse transcription reaction and cDNA amplification (described in "SC3-seq: a method for highly parallel and quantitative measurement of single-cell gene expression. Nucleic Acids Res. 43, e60. (2015)"). The amplified cDNA was used for qPCR using Power SYBR Green PCR Master Mix (Applied Biosystems, 4367659) with a CFX384 Touch Real-Time PCR analysis system (Bio-Rad Laboratories).

### DNA Dot Blot Method

Genomic DNA was extracted from cells using NucleoSpin Tissue (MACHEREY-NAGEL, U0952S) according to the instructions. The extracted DNA was diluted to 5 ng/ml. 0.5 µl of 0.5 N NaOH was added to 20 µl of DNA solution. Next, the mixture was incubated at 99°C for 5 minutes. After incubation, 2.5 µl of 6.6 M ammonium acetate was added. Finally, 2.75 µl of the mixture (corresponding to 50 ng of genomic DNA) was spotted onto a nitrocellulose membrane pre-soaked in 20x SSC. The membrane was heated at 80°C for 2 hours, then incubated in blocking buffer (PBST (PBS containing 0.1% Tween 20) containing 10% skim milk (BD, 232100) and 1% BSA) at room temperature for 1 hour. Next, the membrane was incubated with rabbit anti-5mC antibody (Sigma, SAB5600040) diluted 1:1000 in blocking buffer at 4°C overnight. The membrane was washed 3 times for 5 minutes with PBST, and incubated with HRP-conjugated anti-rabbit IgG goat IgG (Sigma, A6154) diluted 1:8000 in blocking buffer at room temperature for 1 hour. The membrane was washed 3 times for 5 minutes with PBST, and chemofluorescence detection was performed with Chemi-Lumi One Super (Nacalai Tesque, 02230-30) or Chemi-Lumi One Ultra (Nacalai Tesque, 11644-24).

### RNA Sequencing (RNA-seq)

The amplified cDNA described above was used for bulk RNA sequencing. Sequence libraries were prepared similarly to previous reports ("In Vitro Derivation and Propagation of Spermatogonial Stem Cell Activity from Mouse Pluripotent Stem Cells. Cell reports 17, 2789-2804. (2016)", "Long-term expansion with germline potential of human primordial germ cell-like cells in vitro. EMBO J in press. (2020)", "SC3-seq: a method for highly parallel and quantitative measurement of single-cell gene expression. Nucleic Acids Res 43, e60. (2015)"). All reads were processed with Cutadapt v1.18 to trim adapters and poly-A, and mapped to the GRCh38.p12 transcript reference using TopHat2 v2.1.1 90. The resulting BAM files were processed with HTseq v0.9.1 92 to calculate the number of reads per gene. The number of reads per gene was divided by the total number of reads for all chromosomal genes to estimate the RPM value of the gene.

### Single-cell RNA-seq (scRNA-seq)

Cultured cells were harvested and subjected to Cell Multiplexing Oligo labeling. Harvested cells were suspended in Cell Multiplexing Oligo solution and incubated at room temperature for 5 minutes. After incubation, cells were washed with 1% BSA (Sigma, A1519)-PBS(-) and collected by centrifugation at 200 g for 10 minutes. The collected cells were resuspended in 1% BSA-PBS(-) containing anti-TRA-1-85 antibody (BD Bioscience, 563302, 1:20 dilution) and placed on ice for 30 minutes in the dark. Thereafter, cells were washed once with PBS and resuspended in 1% BSA-PBS(-) containing 3 µM DRAQ7 (abcam, ab109202). To isolate live human cells, TRA-1-85(+) DRAQ7(-) cells were sorted into DMEM/F12 (Gibco, 10565-018) + 1% BSA using FACS Aria III. Sorted cells were used for subsequent library construction using Chromium Single Cell 3' Reagent Kit (v3.1 Chemistry Dual Index), followed by sequencing on the Illumina NovaSeq 6000 platform. All steps were performed according to the manufacturer's instructions. Publicly available 10x single-cell RNA-seq data 41, 42, 66 were downloaded and subjected to subsequent processing steps. The obtained read data was processed with CellRanger v6.0.1 with default settings. Read data obtained from samples labeled with Cell Multiplexing Oligo was processed with CellRanger v6.0.1 with the "multi" option.

### Enzymatic Methyl Sequencing (EM-seq)

EM-seq libraries were prepared on the Illumina NovaSeq 6000 platform similarly to the method described for scRNA-seq above and sequenced. Paired-end reads were processed with Trim_Galore! v0.6.3 and mapped onto the GRCh38.p12 genome using Bismark v0.22.1 94 and Bowtie2 v2.3.4.1 (with "-X2000" option). BAM files were processed with the deduplicate_bismark script to remove PCR duplicates, and processed with the bismark_methylation_extractor script to count methyl and non-methyl-cytosines for each CpG or CpA sequence. PBAT data of human sperm, oocytes, blastocysts, human fetal germ cells (9 wpf female germ cells and male germ cells), and hPGCLC-induced cells in xrOvaries (ag120 AG and ag120 AGVT) were processed with Trim_Galore! and Bismark with the "--pbat" option, followed by processing with the bismark_methylation_extractor script as previously described. The mean CpG level of binned loci was calculated as the average of %mC of CpGs (depth >= 4), and only bins with CpG >= 4 were used for analysis. The mean CpA level of binned loci was calculated as [sum of mC calls] exceeding [sum of mC calls and C calls] (sum of mC calls and C calls >= 10). To obtain the mean methylation level of replicates, bins with a read depth of 3 or more in all replicates were used for calculation.

### <Example 1>

hPGCLCs on day 6 induced to differentiate from 585B1-AGDT hiPSCs, which are male hiPSCs having TFAP2C-eGFP (AG) and DAZL-tdTomato (DT) reporter alleles, according to the above "Differentiation induction to Human Primordial Germ Cell-Like Cells (hPGCLCs)", were cultured according to the above "Differentiation induction to Oogonia or Prospermatogonia" under conditions where 25 ng/ml BMP2 was added until day 12 when the first passage was performed, and 100 ng/ml BMP2 was added thereafter. During culture, hPGCLC-derived cells showed stable proliferation, and increased expression of DT was observed by day 32 (c32) from the start of culture. Thereafter, hPGCLC-derived cells constantly increased with a gradual increase in the ratio of cells showing AG positive (AG+) and DT positive (DT+), and surprisingly, almost all cells became AG+DT+ cells by day 140 (c140) (Fig. 2, middle row). At this stage, the total increase was about 10^10 times. In addition, as a control group, culture was also performed without BMP2 (no BMP2) (Fig. 2, upper row). In no BMP2, increased expression of DT was not observed.

Morphologically, both AG+DT- cells and AG+DT+ cells exhibited a spindle shape with a large oval nucleus indicating their motile phenotype, and formed loosely packed colonies with no clear separation between AG+DT- cells and AG+DT+ cells (Fig. 3, left column). Both AG+DT- cells and AG+DT+ cells had normal karyotypes. Notably, the appearance of AG-DT+ cells was observed from around day 82 (c82), which proliferated to 10% or less, but showed no further proliferation thereafter.

Next, hPGCLCs on day 6 induced to differentiate from NCLCN-AGVT hiPSCs, which are female hiPSCs having AG and DDX4 (human VASA homolog)-tdTomato (VT) reporter alleles, according to the above "Differentiation induction to Human Primordial Germ Cell-Like Cells (hPGCLCs)", were cultured according to the above "Differentiation induction to Oogonia or Prospermatogonia" under conditions where 50 ng/ml BMP2 was added until day 12 when the first passage was performed, and 100 ng/ml BMP2 was added thereafter. Under these conditions, hPGCLC-derived cells showed stable proliferation, and increased expression of VT was observed by day 40 (c40) from the start of culture. Thereafter, hPGCLC-derived cells constantly increased with a gradual increase in the ratio of cells showing AG positive (AG+) and VT positive (VT+), and 90% or more of the cells became AG+VT+ cells after day 129 (c129) (Fig. 2, lower row). On day 141 (c141), the total increase was about 10^10 times.

In addition, hPGCLCs derived from 1383D6 hiPSCs having no reporter allele were subjected to the above "Differentiation induction to Oogonia or Prospermatogonia" under similar conditions, and expression of TFAP2C and DDX4 was analyzed by immunostaining. The results are shown in Figure 4. More than half of TFAP2C positive cells expressed DDX4 after 54 days of culture, and more than 95% of TFAP2C positive cells became double positive for TFAP2C and DDX4 on day 93.

Female AG+VT- cells and AG+VT+ cells also resembled each other and male AG+DT- cells and AG+DT+ cells morphologically, forming loosely packed colonies with no clear separation between AG+VT- cells and AG+VT+ cells (Fig. 3, right column). Similarly, the appearance of AG-VT+ cells was also seen from around day 82 (c82), which subsequently accounted for a minority (5% or less) of hPGCLC-derived cells.

From qPCR results, AG+DT+ cells and AG+VT+ cells showed increased expression of major epigenetic reprogramming activation genes (ER genes) (Fig. 5) and showed a significant decrease in 5mC levels compared to hiPSCs and hPGCLCs (DNA dot blot method: Fig. 6), consistent with the view that male AG+DT+ cells and female AG+VT+ cells are mitotic prospermatogonia or oogonia, respectively.

Each of the hPGCLCs on day 6 induced to differentiate from 5 strains of iPSCs: 585B1-AGDT hiPSC, NCLCN-AGVT hiPSC, 585B1-AGVT hiPSC which is a male hiPSC having AG and VT reporter alleles, 1390G3-AGVT hiPSC which is a female hiPSC having AG and VT reporter alleles, and 1383D6 hiPSC, according to the above "Differentiation induction to Human Primordial Germ Cell-Like Cells (hPGCLCs)", were subjected to the first passage according to the above "Differentiation induction to Oogonia or Prospermatogonia". Culture was performed under conditions where BMP2 was added at 25 ng/mL for male strains and 50 ng/ml for female strains until day 12, and 100 ng/ml BMP2 was added thereafter. The proliferation rate of fluorescent reporter/surface antigen marker-positive cells of each strain is shown in Figure 7.

In addition, hPGCLCs on day 6 induced to differentiate from NCLCN-AGVT hiPSCs according to the above "Differentiation induction to Human Primordial Germ Cell-Like Cells (hPGCLCs)" were cultured according to the above "Differentiation induction to Oogonia or Prospermatogonia" under two conditions: adding 50 ng/ml BMP2, or adding 50 ng/ml BMP4 instead of BMP2. The proliferation rate of fluorescent reporter-positive cells under each condition is shown in Figure 8. Further, when fluorescent reporter expression was analyzed, at day 80 of culture, under the condition with BMP2 added, AG+ was 20.1%, AG+VT+ was 74.5%, and VT+ was 5.4%, and under the condition with BMP4 added, AG+ was 13.7%, AG+VT+ was 78.7%, and VT+ was 7.6%. That is, mitotic oogonia were obtained with comparable efficiency regardless of whether BMP2 or BMP4 was used as the BMP receptor type 2 signal activator. Therefore, it was suggested that oogonia or prospermatogonia can be obtained regardless of the means of BMP receptor type 2 signal activation.

Furthermore, 585B1-AGDT was cultured under FBS(-) medium conditions, which is a medium obtained by removing FBS from the medium for differentiation induction to oogonia or prospermatogonia. An increase in AG+DT+ cells, i.e., differentiation into prospermatogonia, was observed using FBS(-) medium, comparable to the medium conditions of the medium for differentiation induction to oogonia or prospermatogonia (with FBS; FBS(+)) (Fig. 9).

The properties of 585B1-AGDT hPGCLC-derived cells (AG+DT- cells and AG+DT+ cells) and NCLCN-AGVT hPGCLC-derived cells (AG+VT- cells, AG+VT+ cells, and AG-VT+ cells) cultured in a medium for differentiation induction to oogonia or prospermatogonia containing BMP were analyzed in detail in Examples 2 and 3.

### <Example 2>

RNA sequencing was performed on iPSCs, iMeLCs, and hPGCLCs, and 585B1-AGDT and NCLCN-AGVT cells cultured for various days in a medium for differentiation induction to oogonia or prospermatogonia, where reporter expression was AG+DT- cells, AG+DT+ cells, or AG+VT- cells, AG+VT+ cells, AG-VT+ cells, respectively, and in addition, 585B1-AGDT cells cultured using a medium for differentiation induction to oogonia or prospermatogonia from which only BMP2 was excluded (BMP(-)). The results were compared with data of hPGCLC differentiation induction in xenogeneic reconstituted ovaries (xrOvaries) using somatic cells of mouse embryonic ovaries (see "Generation of human oogonia from induced pluripotent stem cells in vitro. Science 362, 356-360. (2018)."), and in vivo mitotic pre-spermatogonia/oogonia (see "A Unique Gene Regulatory Network Resets the Human Germline Epigenome for Development. Cell 161, 1453-1467. (2015)."). The results are shown in Figure 10.

In response to BMP2, hPGCLC-derived cells, AG+DT- cells, AG+DT+ cells, or AG+VT- cells, AG+VT+ cells, showed gradually increased ER gene expression while maintaining pluripotency genes and early PGC genes, whereas meiotic genes did not increase in expression. The expression profiles of these major gene groups were very similar between males and females to those of differentiation of hPGCLCs into oogonia in xrOvaries, and more importantly, during in vivo prospermatogonia/oogonia differentiation.

The results of principal component analysis (PCA) on gene expression data are shown in Figure 11. The PCA results show that BMP signaling promotes transcriptome maturation of hPGCLC-derived cells, and this maturation is similar between males and females and shares significant similarities with oogonia differentiation in xrOvaries. Thus, BMP-induced differentiation of hPGCLCs recapitulates oogonia differentiation in xrOvaries and likely differentiation into mitotic prospermatogonia/oogonia in vivo.

To further investigate the relationship between BMP-induced hPGCLC differentiation and in vivo human primordial germ cell (hPGC) differentiation, 10x Chromium-based single-cell RNA-seq (scRNA-seq) analysis was performed on culture day 11 (c11), day 56 (c56), day 86 (c86), and day 117 (c117) in the above "Differentiation induction to Oogonia or Prospermatogonia" of female hPGCLCs, and the analysis data was compared and analyzed with data of in vivo oogonia and fetal eggs in meiotic prophase I at 7 to 16 wpf. By dimensionality reduction using Uniform manifold approximation and projection (UMAP) and Louvain clustering, in vitro and in vivo cells were classified into 10 clusters. Based on the expression of major markers, they were annotated as very early mitosis (VEM) 1/2, early mitosis (EM) 1/2, mitosis (M) 1/2/3, pre-leptotene/leptotene (PLL1/2), and zygotene/pachytene/diplotene (ZPD). The results are shown in Figure 12. VEM cells formed a distinct cluster consisting almost exclusively of c11 AG+VT- cells, and VEM 1/2 represent cells in G1/S and G2/M phases of the cell cycle, respectively. EM consisted mainly of c56 AG+VT- cells and a small number of cells at 7/9/10 wpf where increased expression of oogonia markers such as PRAME, DAZL, DDX4 began to be seen, and EM 1/2 represent cells in G1 phase (in vitro/in vivo) and G1/S/G2/M phases (in vitro) of the cell cycle, respectively. Thus, EM captured early oogonia in vitro and in vivo. M cells consisted of c56/86/117 AG+VT+ cells and oogonia at 7-16 wpf in vivo, and M 1/2/3 represent cells in G1/S, S/G2/M, and G2/M phases of the cell cycle, respectively. The long-term presence (~9 weeks) of M cells both in vitro and in vivo is consistent with the idea that M cells represent self-renewing oogonia. PLL consisted of c86/117 AG-VT+ cells and 9-16 wpf cells, and PLL 1/2 represent cells in G2/M/mitotic phase and G1 phase (in vitro) of the cell cycle, respectively. ZPD consisted mainly of cells at 13/16 wpf in vivo.

### <Example 3>

Enzymatic methyl sequencing (EM-seq) was performed on the genome-wide DNA methylation profiles of hiPSCs, hPGCLCs, and hPGCLCs cultured in a medium for differentiation induction to oogonia or prospermatogonia. In addition, long-read whole genome sequencing was performed on female NCLCN-AGVT and 1390G3 AGVT #526 hiPSC strains using Oxford Nanopore Technology (ONT), and X chromosome allelic sequences were reconstructed based on DNA methylation profiles of phased blocks co-determined by ONT. This dataset was compared with that of in vivo mitotic prospermatogonia/oogonia and hPGCLC-induced cells in xrOvaries. The results are shown in Figures 13 and 14.

The whole autosome 5mC level was about 85% for both male and female hiPSCs, but gradually decreased by hPGCLC differentiation with the differentiation induction medium containing BMP, decreasing to about 10% in c122 male AG+DT+ cells and c127 female AG+VT+ cells (Fig. 13). This indicates a decrease to a level equivalent to 7-9 wpf of in vivo mitotic prospermatogonia/oogonia, which was at an even lower level than hPGCLC-induced cells in xrOvaries. Comparing culture without BMP2 and with BMP2, genome-wide DNA demethylation was more advanced in culture with BMP2. DNA demethylation occurred in all unique elements. Also, as shown in Figure 14, DNA demethylation also occurred in methylated regions (DMRs) of imprinted genes, and in c122 male AG+DT+/c127 female AG+VT+ cells, almost all imprints were erased regardless of abnormal imprinting of parental hiPSCs. "Escapees" that avoided DNA demethylation consisted mainly of evolutionarily young human-specific retrotransposons including long interspersed nuclear elements 1 (LINE1), Alu elements, and endogenous retroviruses (ERVs), which were highly overlapping with those in hPGCLC-induced cells in xrOvaries (ag120 AG+VT-/AG+VT+ cells) and in vivo mitotic prospermatogonia/oogonia.

The X chromosome of male hPGCLC-derived cells and the Xa chromosome of female hPGCLC-derived cells also showed chromosome-wide DNA demethylation in a manner very similar to autosomes, and the overall 5mC level of X or Xa chromosomes decreased to about 15% in c122 male AG+DT+ cells and c127 female AG+VT+ cells, which was equivalent to 9 wpf of in vivo mitotic prospermatogonia/oogonia. From the above, it was clarified that hPGCLC differentiation by the differentiation induction medium containing BMP exhibits comprehensive demethylation of whole autosomes and X/Xa chromosomes, reproducing the reprogramming of DNA methylation state in human germ cells.

### <Example 4>

Among the gene group (cluster 3 in Fig. 15) in which gene expression was not observed in hPGCLCs but increased gene expression was observed in hPGCLC differentiation by the medium for differentiation induction to oogonia or prospermatogonia containing BMP, genes whose promoter 5mC level decreased by 50% or more during the process of differentiation from hiPSCs to oogonia and those common to hPGCLC differentiation in xrOvaries were redefined as core ER genes (Fig. 16). As core ER genes, GTSF1, PRAME, MEG3, DAZL, DDX4, SYCP3, PIWIL1, ANHX, SPATA22, IL22RA1, BRDT, LOC102723543, GCSAML-AS1, RBM46, IL12B, C5orf47, OOEP, DDX43, CHCHD2, ZNF736, LOC101926892, DCAF4L2, KCNV2, TUSC1, C9orf64, ANKRD2, LOC105369746, SPESP1, USP6, LOC105376917, ZNF492, TDRD12, ZNF534, NLRP9, ZNF667, ZNF667-AS1, LOC107985433, DSCR8, MAGEB2, MAGEB1, CXorf67, LUZP4, and LDOC1 were found. The redefined core ER genes showed very similar profiles in in vitro and in vivo hPGC(LC) differentiation (Fig. 17). Therefore, expression of these core ER genes can be an example of characteristics possessed by prospermatogonia and/or oogonia.

### Industrial Applicability

According to the present invention, it has become possible to obtain oogonia and prospermatogonia from PGCLCs as a pure cell population by a very simple operation, even without providing mixing with xenogeneic cells such as mouse ovarian cells or testicular cells or cells derived from other individuals. Furthermore, since the oogonia or prospermatogonia obtained in this way as a pure cell population can be cryopreserved as they are, it is also advantageous for subsequent use.

## Claims

1. A method for producing oogonia or prospermatogonia, comprising a step of culturing primordial germ cell-like cells or primordial germ cells in a medium containing a BMP receptor type 2 signal activator to obtain oogonia or prospermatogonia.

2. The method for producing oogonia or prospermatogonia according to claim 1, wherein the BMP receptor type 2 signal activator is one or more selected from the group consisting of BMP2, BMP4, and BMP7.

3. The method for producing oogonia or prospermatogonia according to claim 1, wherein the medium further contains a Wnt signal inhibitor.

4. The method for producing oogonia or prospermatogonia according to claim 3, wherein the Wnt signal inhibitor is IWR1 or XAV939.

5. The method for producing oogonia or prospermatogonia according to claim 1, wherein the medium further contains one or a combination of two or more selected from stem cell factor (SCF), a cAMP enhancer, and basic fibroblast growth factor (bFGF).

6. The method for producing oogonia or prospermatogonia according to claim 1, wherein the culture period is 10 days or more.

7. The method for producing oogonia or prospermatogonia according to claim 1, wherein the oogonia and prospermatogonia are DAZL and DDX4 positive cells.

8. The method for producing oogonia or prospermatogonia according to claim 1, wherein the primordial germ cell-like cells are primordial germ cell-like cells obtained by inducing differentiation of pluripotent stem cells.

9. The method for producing oogonia or prospermatogonia according to claim 1, which does not include a step of providing an external environment that promotes differentiation induction into oogonia or prospermatogonia using xenogeneic ovarian cells or testicular cells, or ovarian cells or testicular cells derived from other individuals.

10. The method for producing oogonia or prospermatogonia according to any one of claims 1 to 9, wherein the primordial germ cell-like cells and primordial germ cells are human primordial germ cell-like cells and human primordial germ cells, and the oogonia and prospermatogonia are human oogonia and human prospermatogonia.

11. A cell population obtained by inducing differentiation of primordial germ cell-like cells or primordial germ cells, comprising 80% or more of oogonia or prospermatogonia.
